(19) Europäisches Patentamt
European Patent Office
Office européen des brevets

(11) Publication number: **0 104 691**
**B1**

(12) **EUROPEAN PATENT SPECIFICATION**

(45) Date of publication of patent specification: **06.07.88**

(21) Application number: **83201250.4**

(22) Date of filing: **30.08.83**

(51) Int. Cl.[4]: **C 07 D 213/55,**
C 07 D 213/56,
C 07 D 213/57,
C 07 D 401/06,
C 07 D 403/06,
C 07 D 405/06,
C 07 D 409/06, A 01 N 43/40

(54) **Fungicidally active compounds.**

(30) Priority: **27.09.82 GB 8227481**

(43) Date of publication of application:
**04.04.84 Bulletin 84/14**

(45) Publication of the grant of the patent:
**06.07.88 Bulletin 88/27**

(84) Designated Contracting States:
**AT BE CH DE FR GB IT LI LU NL SE**

(56) References cited:
**CHEMICAL ABSTRACTS, vol. 69, 1968, page 4097, abstract 43748s, Columbus, Ohio, US; L. KUCZYNSKI et al.: "Synthesis of new derivatives of phenylacetic acid"**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **ten Haken, Pieter**
**Konkelboet The Street**
**Eastling Nr. Faversham Kent (GB)**
Inventor: **Webb, Shirley Beatrice**
**17 North Street Ashford Road**
**Sheldwich Faversham Kent (GB)**

(74) Representative: **Hunter, Keith Roger Ian et al**
**4 York Road**
**London SE1 7NA (GB)**

**EP 0 104 691 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to fungicidally active compounds. Certain ethane derivatives have been disclosed in Diss. Pharm. Pharmacol. 1968, 20 (2), 169—77.

Certain alkane derivatives have been disclosed as fungicides, for example see US Patent Specification No. 3397273. The applicants have now found that certain novel ethane derivatives also have useful fungicidal activity.

The present invention therefore provides a compound of the general formula (I)

$$Ar^1\!-\!\underset{R^3}{\overset{R^1}{C}}\!-\!\underset{H}{\overset{R^2}{C}}\!-\!Ar^2 \qquad (I)$$

or an N-oxide, salt or metal salt complex thereof, wherein one of $Ar^1$ and $Ar^2$ represents an unsubstituted or substituted 6 membered heteroaromatic ring containing 1 or 2 nitrogen atoms and the other of $Ar^1$ and $Ar^2$ represents an unsubstituted or substituted 5 or 6 membered heterocyclic ring having 1 or 2 heteroatoms selected from nitrogen, oxygen and sulphur atoms or an unsubstituted or substituted phenyl group; $R^1$ represents a hydrogen atom or an unsubstituted or substituted $C_1$—$C_6$ alkyl group; $R^2$ represents a cyano group, a —COOH group or a $C_{1-6}$ alkyl ester or a $C_{1-6}$ alkyl thioester thereof, or a group

$$-C(=O)-N(R^4)(R^5) \quad \text{or} \quad -C(=O)-Y$$

wherein each of $R^4$ and $R^5$ independently represents a hydrogen atom or an unsubstituted or substituted $C_{1-6}$ alkyl or phenyl group or $R^4$ and $R^5$ together with the interjacent nitrogen atom represent a 5 or 6 membered heterocyclic ring containing up to one heteroatom selected from oxygen and nitrogen atoms in addition to the interjacent nitrogen atom and Y represents a halogen atom; $R^3$ represents a cyano group, a COOH group or a $C_{1-6}$ alkyl ester or a $C_{1-6}$ alkyl thioester thereof, a hydroxy or thio group, or a group

$$OR^6,\ -SR^6,\ -\overset{O}{\overset{\|}{S}}-R^6,\ -\underset{O}{\underset{\|}{\overset{O}{\overset{\|}{S}}}}-R^6,\ -OR^7,\ -SR^7,\ -OCONR^8R^9 \text{ or } -NR^8R^9$$

wherein $R^6$ represents an unsubstituted or substituted $C_{1-6}$ alkyl or phenyl group, $R^7$ represents a $C_{1-6}$ alkanoyl benzoyl or toluyl group and $R^8$ and $R^9$ are as defined above for $R^4$ and $R^5$, and wherein the substituents of substituted aliphatic groups are selected from halogen atoms, $C_{1-6}$ alkoxy, $C_{1-6}$ alkylthio, cyano carboxy, $C_{1-6}$ alkoxycarbonyl formyloxy, methylcarbonyloxy, methylaminocarbonyloxy, hydroxy, amino and $C_{3-6}$ cycloalkyl groups and unsubstituted or substituted phenyl or phenoxy groups; and the substituents of substituted phenyl, phenoxy and heterocyclic groups are selected from halogen atoms, hydroxy, methylenedioxy, amino, cyano, $C_{1-6}$ alkylsulphonyl and nitro groups and unsubstituted or substituted $C_{1-6}$ alkyl, $C_{2-6}$ alkenyl, $C_{2-6}$ alkynyl, $C_{1-6}$ alkoxy, $C_{1-6}$ alkylthio, $C_{1-6}$ alkoxycarbonyl, phenyl and phenoxy groups, with the proviso that, when $Ar^2$ represents an unsubstitued phenyl group, $R^1$ represents a hydrogen atom, $R^2$ represents —$CONH_2$ and $R^3$ represents —$NH_2$, then $Ar^1$ does not represent an unsubstituted 2-, 3- or 4-pyridyl group.

Throughout this specification, unless otherwise stated, any aliphatic moiety present preferably contains up to 6, especially up to 4, carbon atoms.

A phenyl group $Ar^1$ or $Ar^2$ is preferably unsubstituted or substituted by up to 3 of the same or different substituents given above. Most preferably it is unsubstituted, monosubstituted or disubstituted. Preferred substituents are selected from halogen atoms and nitro, cyano, alkyl, haloalkyl, alkoxy, haloalkoxy and alkylthio groups. Especially preferred substituents are 1 or 2 halogen atoms.

As noted above one or both of $Ar^1$ and $Ar^2$ represents an unsubstituted or substituted 6 membered heteroaromatic ring having 1 or 2 nitrogen atoms. These groups are unsubstituted or substituted pyridyl, pyrazinyl, pyridazinyl and pyrimidinyl groups.

The other of $Ar^1$ and $Ar^2$ may represent an unsubstituted or substituted 5 or 6 membered heterocyclic ring having 1 or 2 heteroatoms. Selected from nitrogen, oxygen and sulphur atoms. Suitable rings include

those listed above and also thiophene, furan, pyrrole, pyrazole, pyrrolidine, imidazole and the corresponding partially or fully saturated analogues. Thiophene and furan groups are especially suitable.

A heterocyclic group $Ar^1$ or $Ar^2$ may be substituted by one or more of the substituents given above for a phenyl group; preferably however it is unsubstituted or substituted by one or more alkyl groups or halogen atoms; most preferably such a group is unsubstituted.

Preferred compounds of the general formula I and N-oxides, salts and complexes thereof, are those in which one of $Ar^1$ and $Ar^2$ represents an unsubstituted pyrazinyl or, especially, an unsubstituted pyridyl group and the other of $Ar^1$ and $Ar^2$ represents an unsubstituted pyridyl group or an unsubstituted or substituted phenyl group; more preferably one of $Ar^1$ and $Ar^2$ is an unsubstituted 3-pyridyl group and the other of $Ar^1$ and $Ar^2$ is a halo- substituted phenyl group for example a mono or di-chloro or fluoro substituted phenyl group, preferably a dichloro substituted phenyl group.

Preferably $R^1$ represents a methyl group or, especially, a hydrogen atom.

If $R^2$ or $R^3$ represents an ester or thioester of —COOH, this ester or thioester is derived from an unsubstituted alkyl alcohol or thiol having up to 6 carbon atoms.

If $R^4$ and/or $R^5$ represent a substituted $C_{1-6}$ alkyl or phenyl group, the preferred substituents are, for example, those given above. For example, a phenyl group $R^4$ and/or $R^5$ may be substituted as described above for $Ar^1$ or $Ar^2$.

If $R^4$ and $R^5$ together with the interjacent nitrogen atom represent a 5 or 6 membered heterocyclic ring as defined above, this ring may be saturated or unsaturated.

Preferably $R^2$ represents a cyano group, a —COOH group or a $C_{1-6}$ alkyl ester or a $C_{1-6}$ alkyl thioester thereof, or a group

$$-C(=O)-N(R^4)(R^5) \quad \text{or} \quad -C(=O)-Cl$$

wherein each of $R^4$ and $R^5$ independently represents a hydrogen atom, an unsubstituted $C_{1-6}$ alkyl group or an unsubstituted or substituted phenyl group, or $R^4$ and $R^5$ together with the interjacent nitrogen atom represent a pyrrolidone or piperidine ring.

Most preferably $R^2$ is a —COOH group or a $C_{1-6}$ alkyl ester or a $C_{1-6}$ alkyl thioester thereof.

Suitable carboxylic acids from which an alkanoyl group $R^7$ is derived include alkanoic acids such as acetic acid, propanoic acid and formic acid.

Preferably $R^3$ represents a cyano group, a hydroxy group or a group

$$-OR^6,\ -SR^6,\ -S(=O)-R^6,\ -S(=O)_2-R^6,\ -OR^7,\ -SR^7 \text{ or } -NR^8R^9$$

wherein $R^6$ represents an unsubstituted or substituted $C_{1-6}$ alkyl group or an unsubstituted or substituted phenyl group; $R^7$ represents an unsubstituted $C_{1-6}$ alkanoyl group; benzoyl or toluyl group; and each of $R^8$ and $R^9$ has one of the preferred meanings given above for $R^4$ and $R^5$.

Most preferably $R^3$ represents a hydroxy group or a group

$$-OR^6,\ -SR^6, \text{ or } -S(=O)_2-R^6$$

wherein $R^6$ represents a $C_{1-4}$ alkyl group or a phenyl group which is unsubstituted or substituted by one or more halogen atoms.

As stated above, the invention includes metal salt complexes and salts of compounds of the general formula I. Suitable salts include salts with sulphonic acids, for example benzene- or toluenesulphonic acid, carboxylic acids for example tartaric or acetic acid, or inorganic acids for example the hydrohalic acids or sulphuric acid. If $R^2$ or $R^3$ represents a —COOH group, a salt may be a metal salt, for example an alkali or alkaline earth metal salt, the ammonium salt, or a substituted ammonium salt, for example, an alkyl-substituted ammonium salt. Suitable metal salts which form complexes with compounds of the general formula I include those of heavy metals such as iron, copper, zinc and manganese, in which the anions may, for example, be derived from one of those acids given above.

It will be appreciated that both carbon atoms of the ethane base unit are asymmetric and give rise to isomeric forms of compounds of general formula I. Other possibilities for isomerism may arise depending on the specific groups present in the molecule. The invention encompasses all individual isomers as well as mixtures of isomers.

The invention further provides a process for the preparation of a compound according to the invention, which comprises reacting a compound of the general formula

$$Ar^1R^1C{=}CR^{10}Ar^2 \qquad (II)$$

in which $Ar^1$, $R^1$ and $Ar^2$ have the meanings given for the general formula I, and $R^{10}$ represents a —COOH group or an ester or thioester thereof, with a compound of the general formula $HR^{11}$, in which $R^{11}$ represents a cyano, hydroxy or thio group or a group of formula —$OR^6$, —$SR^6$ or —$NR^8R^9$ where $R^6$, $R^8$ and $R^9$ have the meanings given for the general formula I; and if desired, converting the resulting compound of the invention into any other compound of the invention.

In general, the process according to the invention leads to a mixture of isomers of the compound of general formula I. As noted above both carbon atoms of the ethane base unit are asymmetric. This asymmetry alone gives rise to various different stereoisomers whose absolute configuration may be defined in terms of the Cahn-Ingold-Prelog convention [see Roberts and Caserio, Basic Principles of Organic Chemistry, p. 529, (1965)]. Using this convention, isomers RR and SS are enantiomorphs with identical physical properties which usually prevents separation by physical methods; similarly for the isomers RS and SR.

However, the isomers RR and RS are diastereoisomers with different physical properties which enable separation by conventional means; this is also the case for the isomers SS and SR.

Furthermore, as noted above, other possibilities for isomerism may arise depending on the specific groups present in the molecule. These isomers may also differ in their physical properties.

Hence, certain of the reaction products may be separated by conventional physical means, for example using chromatography. When separating a mixture of two isomer pairs, one isomer pair will usually be eluted faster than the other pair.

The molar ratio of the reactants used in the process according to the invention is not critical, and may for example be in the range of from 5:1 to 1:5, especially 2:1 to 1:2.

The reaction is suitably carried out in the presence of a solvent; typical solvents are for example alcohols such as methanol or ethanol; ethers such as tetrahydrofuran or dimethoxyethane; chlorinated hydrocarbons such as methylene chloride; anhydrides such as acetic anhydride; esters such as ethyl acetate; amides such as dimethylformamide or dimethyl acetamide; ketones such as acetone, dimethyl ketone or methyl ethyl ketone; and nitro-alkanes such as nitromethane. Mixtures of solvents may be suitable.

The reaction is preferably carried out in the presence of a base. Suitable basis include primary, secondary or tertiary amines, for example, triethylamine or the secondary cyclic amine piperidine; alkali metal hydrides, amides or alkoxides, for example sodium ethoxide; and alkali metal and alkaline earth metal hydroxides.

The reaction is preferably carried out in an inert atmosphere, for example, under nitrogen, especially when $R^3$ contains a sulphur atom.

The temperature of the reaction is suitably in the range of from 0° to 180°C. It may in some cases be convenient to carry out the reaction at the reflux temperature of the reaction mixture.

The direct product resulting from the reaction of the compound II with the compound $HR^{11}$, is a compound of the general formula I in which $R^2$ represents a —COOH group or an ester or thioester thereof, and $R^3$ has one of the meanings given for $R^{11}$. As stated above, this resulting compound can be converted to any other compound of the invention as desired, generally by methods analogous to known methods. For example a hydroxy or thio group $R^3$ can be converted into a group —$OR^6$, —$SR^6$, —$OR^7$ or —$SR^7$ by reaction with a compound of the general formula $HalR^6$ or $HalR^7$ where Hal represents a halogen atom, or, where appropriate, by reaction with an olefin. A cyano group $R^3$ can be converted into a —$CONH_2$ or —COOH group by hydrolysis, or into an ester group by alcoholysis. A —COOH group $R^2$ or $R^3$ may be esterified by reaction with the appropriate alcohol or thiol, or converted into the acid halide —COY by reaction with a halogenating agent, for example phosphorous pentachloride or thionyl chloride. The acid halide may be converted into an ester or thioester by reaction with the appropriate alcohol or thiol, or into a substituted amide by reaction with an amine $HNR^4R^5$ or $NHR^8R^9$. A —COOH group $R^2$ or $R^3$ can be converted into a cyano group by dehydration, for example using phosphorus pentoxide, an —$SR^6$ group $R^3$ may be oxidised to give the corresponding $R^3$ groups —$SOR^6$ or —$SO_2R^6$. Suitable oxidising agents include, for example, hydrogen peroxide, benzoyl peroxide, and peroxy acids.

A resulting compound of formula I may be converted into its N-oxide or a salt or a metal salt complex thereof by methods analogous to known methods, for example by reaction with an oxidising agent or with the appropriate acid, base or salt. A resulting salt can be converted into the free compound by reaction with an acid binding agent or an acid, as appropriate.

The starting material of the general formula II may be prepared by reacting a compound of the general formula III

$$Ar^1—\overset{\overset{\displaystyle O}{\|}}{C}—R^1 \qquad (III)$$

with a compound of general formula (IV)

$$Ar^2—CH_2—R^{10} \qquad (IV)$$

wherein $Ar^1$, $Ar^2$, $R^1$ and $R^{10}$ have the meanings given for the compounds of the general formula II.

The conditions under which this reaction may be carried out are described in our copending application EP—A—104690.

The compounds of the invention have been found to show interesting activity as fungicides. Accordingly the invention provides a fungicidal composition comprising a compound of the general formula I as defined above or an N-oxide, salt or metal salt complex thereof in association with at least one carrier and a method of making such a composition which comprises bringing a compound of general formula I as defined above or an N-oxide, salt or metal salt complex into association with at least one carrier.

The invention further provides a method of controlling fungus at a locus, which comprises applying to the locus a compound or composition according to the invention. Suitable dosages are, for example, in the range of from 0.05 to 4 kg active material per hectare. The method of the invention is especially useful for the treatment or prevention of fungal attack in seeds, soil or plants; crops susceptible to powdery mildews, for example cereals or apples, may for example be treated.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example, isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3—10% w of a dispersing agent, and whereby necessary, 0—10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½—10% w of active ingredient. Granules are usually

prepared to have a size between 10 and 100 bS mesh (1.676—0.152 mm), and may be manufactured by agglomeration or impregnation techniques. Generally, granules will contain ½—75% w active ingredient and 0—10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The composition of the invention may also contain other ingredients, for example, other compounds possessing herbicidal, insecticidal or fungicidal properties.

The following Examples illustrate the invention. The terms "A", "B" and "C" refer to the order in which the isomeric reaction products are eluted from the chromatography column, "A" referring to the product eluted first etc.

Example 1

Preparation of methyl 2-(2',4'-dichlorophenyl)-3-(1'-methylpropylthio)-3-(3-pyridyl)propanoic ester

To a stirred solution of methyl 2-(2',4'-dichlorophenyl)-3-(3-pyridyl)propenoic ester (5.7 g) in dry dimethoxyethane (50 ml) and dry methanol (10 ml) under a nitrogen atmosphere was added 1-methylpropane-1-thiol (16.6 g) and piperidine (1.57 g). The mixture was stirred and heated under reflux for 16 hours. After cooling, solvents were removed *in vacuo*, and the residue taken up in ether, washed three times with water and dried with magnesium sulphate. After filtration and removal of the solvent, the residual oil was subjected to column chromatography on silica gel, eluting with ether/hexane (2:1).

Two fractions were eluted. The faster eluted material (A) was obtained in 46% yield as a white solid, melting point 81—82.5°C. The slower eluted material (B) was obtained in a 28% yield as a white solid, melting point 86—91°C.

The following elemental analysis results were obtained:

Product A

| | | | |
|---|---|---|---|
| Calculated: | C: 57.29 | H: 5.28 | N: 3.52 |
| Found: | C: 57.3 | H: 5.3 | N: 3.4 |

Product B

| | | | |
|---|---|---|---|
| Calculated: | C: 57.29 | H: 5.28 | N: 3.52 |
| Found: | C: 57.5 | H: 5.6 | N: 3.6 |

Example 2

Preparation of the HCl salt of the product A of Example 1

Hydrogen chloride gas was bubbled into a stirred solution of product A of Example 1 (1.0 g) in dry ether (25 ml) for about 5 minutes. Nitrogen was then bubbled through the reaction mixture to remove excess hydrogen chloride. The white solid that had separated was filtered off, washed with ether followed by 40/60 petroleum spirit, and dried.

The yield of product was 1.1 g (100%); melting point 177—181°C.

The following elemental analysis results were obtained:—

| | | | |
|---|---|---|---|
| Calculated: | C: 52.47 | H: 5.06 | N: 3.22 |
| Found: | C: 52.6 | H: 5.5 | N: 3.2 |

Example 3

Preparation of ethyl 2-phenyl-3-(4'-chlorophenylthio)-3-(3-pyridyl)propanoic ester

To a stirred solution of ethyl 2-phenyl-3-(3-pyridyl)propenoic ester (1.75 g) in dry dimethoxyethane (25 ml), under nitrogen, was added 4-chlorothiophenol (2 g) followed by a solution of dry piperidine (0.7 ml) in dry dimethoxyethane (10 ml). The reaction mixture was stirred and heated under reflux for 16 hours. After cooling, solvent was removed *in vacuo*, the residue taken up in ether, washed three times with water and dried using magnesium sulphate. After filtration and removal of solvent *in vacuo*, the crude reaction product was subjected to column chromatography on silica gel, eluting with ether-hexane (2:1).

Two fractions were eluted. The faster eluted material (A), a colourless solid, was obtained in 33% yield,

melting point 92—93°C. The slower eluted material (B) was obtained as a colourless solid in a 29% yield, melting point 124—126°C.

The following elemental analysis results were obtained:

Product A

| | | | |
|---|---|---|---|
| Calculated: | C: 66.42 | H: 5.03 | N: 3.52 |
| Found: | C: 66.4 | H: 4.7 | N: 3.7 |

Product B

| | | | |
|---|---|---|---|
| Calculated: | C: 66.42 | H: 5.03 | N: 3.52 |
| Found: | C: 66.4 | H: 4.9 | N: 3.7 |

Example 4

Preparation of methyl 2-(2',4'-dichlorophenyl)-3-(1'-phenylethylsulphonyl)-3-(3-pyridyl)propanoic ester.

Methyl 2-(2',4'-dichlorophenyl)-3-(1'-phenylethylthio)-3-(3-pyridyl)propanoic ester [product A of Example 13—a mixture of two diastereoisomers in a ratio approximately 1:1—2 g] was dissolved in chilled formic acid (16.3 ml). Water (2.06 ml) and 30% hydrogen peroxide (1.5 ml) were added, and the mixture left at 5°C for 66 hours. The reaction mixture was then poured into approximately 300 ml of ice-water. After several hours the colourless solid that had separated was filtered off, washed with water and dried. The crude reaction product was subjected to column chromatography on silica gel, using diethyl ether as an eluant. Two fractions were eluted. The faster eluting material (A) a colourless solid was obtained in 40% yield; melting point 139—141°C. The slower eluted material (B) was obtained in a 19% yield as a colourless solid, melting point 164—167°C.

The following elemental analysis results were obtained:

Product A

| | | | |
|---|---|---|---|
| Calculated: | C: 57.74 | H: 4.39 | N: 2.93 |
| Found: | C: 57.8 | H: 4.5 | N: 3.0 |

Product B

| | | | |
|---|---|---|---|
| Calculated: | C: 57.74 | H: 4.39 | N: 2.93 |
| Found: | C: 57.3 | H: 4.5 | N: 3.0 |

Example 5

Preparation of ethyl 3-(2',4'-dichlorophenyl)-3-hydroxy-2-(3-pyridyl)propanoic acid ester

To a mixture of 2,4-dichlorobenzaldehyde (14.42 g) and ethyl (3-pyridyl) acetic acid ester (13.6 g) in absolute ethanol (80 ml), stirred at 50°C, were added 12.86 ml of a solution of sodium (2.7 g) in absolute ethanol (70 ml). The mixture was left for 16 hours without further heating. The solvent was removed *in vacuo* and the residue taken up in diethyl ether, washed three times with water and dried using magnesium sulphate.

After filtration, the solvent was removed *in vacuo* and the residue subjected to column chromatography on silica gel using diethyl ether as an eluant. The first product to be eluted was ethyl 2-(3-pyridyl)-3-(2',4'-dichlorophenyl)-propenoic acid ester, a colourless solid, mpt. 64—68°C (A).

The second and third fractions (B and C) to be eluted were diastereoisomers of ethyl 3-(2',4'-dichlorophenyl)-3-hydroxy-2-(3-pyridyl)-propanoic acid ester; product B was obtained as a colourless solid, mpt. 126—129°C in a 6% yield and product C; the slower eluted material was obtained as a colourless solid in an 8% yield. These products had been produced by the reaction of ethyl 2-(3-pyridyl)-3-(2',4'-dichlorophenyl)-propenoic acid ester with water *in situ*.

The following elemental analysis results were obtained:

Product B

| | | | |
|---|---|---|---|
| Calculated: | C: 56.47 | H: 4.41 | N: 4.12 |
| Found: | C: 56.3 | H: 4.4 | N: 4.2 |

Product C

| | | | |
|---|---|---|---|
| Calculated: | C: 56.47 | H: 4.41 | N: 4.12 |
| Found: | C: 56.5 | H: 4.5 | N: 4.2 |

Examples 6 to 26

By methods analogous to those described in Examples 1 to 5, the following compounds were prepared.

Analysis and physical data figures are given in Table I.

TABLE I

| Example No. | $Ar^1$ | $Ar^2$ | $R^1$ | $R^2$ | $R^3$ | | Elemental analysis C | H | N | Physical data |
|---|---|---|---|---|---|---|---|---|---|---|
| 6A | 3-pyridyl | 2,4-dichloro-phenyl | H | $-COOCH_3$ | $EtO-C(=O)-CH_2S-$ | Calc.<br>Found. | 53.27<br>53.2 | 4.44<br>4.4 | 3.27<br>3.3 | Mpt: 67—69°C |
| 6B | 3-pyridyl | 2,4-dichloro-phenyl | H | $-COOCH_3$ | $EtO-C(=O)-CH_2S-$ | Calc.<br>Found. | 53.27<br>53.1 | 4.44<br>4.6 | 3.27<br>3.3 | Mpt: 77—78°C |
| 7* copper (II) chloride complex (2 moles of Ethane derivative per mole of $CuCl_2$) | 3-pyridyl | 2,4-dichloro-phenyl | H | $-COOCH_3$ | $EtO-C(=O)-CH_2S-$ | Calc.<br>Found. | 46.04<br>45.7 | 3.84<br>3.9 | 2.83<br>2.8 | Mpt: 153—154°C (with decomposition) |
| 8A | 3-pyridyl | 2,4-dichloro-phenyl | H | $-COOCH_3$ | $(CH_3)_3CS-$ | Calc.<br>Found. | 57.29<br>57.3 | 5.28<br>5.3 | 3.52<br>3.6 | Mpt: 127—129°C |
| 8B | 3-pyridyl | 2,4-dichloro-phenyl | H | $-COOCH_3$ | $(CH_3)_3CS-$ | Calc.<br>Found. | 57.29<br>56.8 | 5.28<br>5.1 | 3.52<br>3.6 | Mpt: 106—112°C |

* Prepared from the compound of Example 6, product A.

TABLE I (cont.).

| Example No. | $Ar^1$ | $Ar^2$ | $R^1$ | $R^2$ | $R^3$ | | Elemental analysis C | H | N | Physical data |
|---|---|---|---|---|---|---|---|---|---|---|
| 9A | 3-pyridyl | 2,4-dichloro-phenyl | H | $—COO(CH_2)_3CH_3$ | $(CH_3)_3CS—$ | Calc.<br>Found. | 60.00<br>60.1 | 6.14<br>6.2 | 3.18<br>3.3 | Mpt: 79—81°C |
| 9B | 3-pyridyl | 2,4-dichloro-phenyl | H | $—COO(CH_2)_3CH_3$ | $(CH_3)_3CS—$ | Calc.<br>Found. | 60.00<br>60.1 | 6.14<br>6.4 | 3.18<br>3.2 | Mpt: 104—106°C |
| 10A | 2,4-dichloro-phenyl | 3-pyridyl | H | $—COO(CH_2)_3CH_3$ | $(CH_3)_3C—S—$ | Calc.<br>Found. | 60.00<br>59.6 | 6.14<br>6.2 | 3.18<br>3.0 | Mpt: 73—75°C |
| 10B | 2,4-dichloro-phenyl | 3-pyridyl | H | $—COO(CH_2)_3CH_3$ | $(CH_3)_3C—S—$ | Calc.<br>Found. | 60.00<br>59.8 | 6.14<br>6.2 | 3.18<br>3.3 | Mpt: 92.5—93.5°C |
| 11A | 3-pyridyl | phenyl | H | $—COOC_2H_5$ | —OH | Calc.<br>Found. | 70.85<br>70.9 | 6.27<br>6.4 | 5.17<br>5.3 | Mpt: 128—133°C |
| 11B | 3-pyridyl | phenyl | H | $—COOC_2H_5$ | —OH | Calc.<br>Found. | 70.85<br>70.9 | 6.27<br>6.3 | 5.17<br>5.3 | Mpt: 163—165°C |

TABLE I (cont.).

| Example No. | Ar[1] | Ar[2] | R[1] | R[2] | R[3] | | Elemental analysis C | H | N | Physical data |
|---|---|---|---|---|---|---|---|---|---|---|
| 12A | 3-pyridyl | 2,4-dichloro-phenyl | H | —$COOCH_3$ | HO—$CH_2CH_2S$— | Calc.<br>Found. | 52.85<br>52.5 | 4.40<br>4.1 | 3.63<br>3.1 | Mpt: 143—149°C |
| 12B | 3-pyridyl | 2,4-dichloro-phenyl | H | —$COOCH_3$ | HO—$CH_2CH_2S$— | Calc.<br>Found. | 52.85<br>52.4 | 4.40<br>4.5 | 3.63<br>3.6 | |
| 13A | 3-pyridyl | 2,4-dichloro-phenyl | H | —$COOCH_3$ | $CH_3$—CH(Ph)—S— | Calc.<br>Found. | 61.88<br>61.5 | 4.71<br>4.7 | 3.14<br>3.1 | Mpt: 94—100°C |
| 13B | 3-pyridyl | 2,4-dichloro-phenyl | H | —$COOCH_3$ | $CH_3$—CH(Ph)—S— | Calc.<br>Found. | 61.88<br>62.8 | 4.71<br>5.3 | 3.14<br>3.0 | Mpt: 64—76°C |
| 13C | 3-pyridyl | 2,4-dichloro-phenyl | H | —$COOCH_3$ | $CH_3$—CH(Ph)—S— | Calc.<br>Found. | 61.88<br>62.0 | 4.71<br>4.7 | 3.14<br>3.1 | Mpt: 107—109°C |
| 14* | 3-pyridyl | 2,4-dichloro-phenyl | H | —$COOCH_3$ | $CH_3$—$CH_2$—CH($CH_3$)—S(=O)(=O)— | Calc. | 53.02 | 4.88 | 3.26 | Mpt: 118—121°C |
| 15A | 3-pyridyl | 3-pyridyl | H | —$COOCH_3$ | $(CH_3)_3CS$— | Calc.<br>Found. | 65.45<br>65.6 | 6.67<br>6.7 | 8.48<br>8.7 | Mpt: 111—116°C |
| 15B | 3-pyridyl | 3-pyridyl | H | —$COOCH_3$ | $(CH_3)_3CS$— | Calc.<br>Found. | 65.45<br>65.6 | 6.67<br>7.0 | 8.48<br>8.5 | Mpt: 122—123°C |

* prepared from the compound of Example 1 product A.

TABLE I (cont.).

| Example No. | $Ar^1$ | $Ar^2$ | $R^1$ | $R^2$ | $R^3$ | | Elemental analysis C | H | N | Physical data |
|---|---|---|---|---|---|---|---|---|---|---|
| 16A | 3-pyridyl | 3-pyridyl | H | $-COOCH_3$ | $(CH_3)_3C-S-$ | Calc.<br>Found. | 65.45<br>65.6 | 6.67<br>6.7 | 8.48<br>8.7 | Mpt: 111—116°C |
| 16B | 3-pyridyl | 3-pyridyl | H | $-COOCH_3$ | $(CH_3)_3C-S-$ | Calc.<br>Found. | 65.45<br>65.6 | 6.67<br>7.0 | 8.48<br>8.5 | Mpt: 122—123°C |
| 17 | 2,4-dichloro-phenyl | 3-pyridyl | H | $-COOCH_3$ | $(CH_3)_3C-S-$ | Calc.<br>Found. | 57.29<br>56.7 | 5.28<br>5.3 | 3.52<br>3.4 | oil |
| 18 | 2,4-dichloro-phenyl | 3-pyridyl | H | CN | $(CH_3)_2CHCH_2-S-$ | Calc.<br>Found. | 59.18<br>58.7 | 4.93<br>4.6 | 7.67<br>7.5 | Mpt: 103—105°C |
| 19 | 2,4-dichloro-phenyl | 3-pyridyl | H | $-CONHC(CH_3)_3$ | $(CH_3)_2CHCH_2-S-$ | Calc.<br>Found. | 60.14<br>59.8 | 6.38<br>6.1 | 6.38<br>6.2 | Mpt: 118—121°C |
| 20A | 2,4-dichloro-phenyl | 3-pyridyl | H | $-CONHCH_2C(=O)OEt$ | $(CH_3)_2CHCH_2-S-$ | Calc.<br>Found. | 56.29<br>56.2 | 5.54<br>5.2 | 5.97<br>6.2 | Mpt: 117—119°C |
| 20B | 2,4-dichloro-phenyl | 3-pyridyl | H | $-CONHCH_2C(=O)OEt$ | $(CH_3)_2CHCH_2-S-$ | Calc.<br>Found. | 56.29<br>56.0 | 5.54<br>5.4 | 5.97<br>5.5 | Purity 70% |

TABLE I (cont.).

| Example No. | $Ar^1$ | $Ar^2$ | $R^1$ | $R^2$ | $R^3$ | Elemental analysis | C | H | N | Physical data |
|---|---|---|---|---|---|---|---|---|---|---|
| 21A | 2,4-dichloro-phenyl | 3-pyridyl | H | $-CONH(CH_2)_2OC(=O)CH_3$ | $(CH_3)_2CHCH_2-S-$ | Calc.<br>Found. | 56.28<br>56.3 | 5.54<br>5.5 | 5.97<br>5.8 | Mpt: 101—103°C |
| 21B | 2,4-dichloro-phenyl | 3-pyridyl | H | $-CONH(CH_2)_2OC(=O)CH_3$ | $(CH_3)_2CHCH_2-S-$ | Calc.<br>Found. | 56.28<br>56.3 | 5.54<br>5.5 | 5.97<br>5.8 | Mpt: 130—131°C |
| 22A | 2,4-dichloro-phenyl | 3-pyridyl | H | —COOEt | $-OC(=O)NHCH_3$ | Calc.<br>Found. | 54.41<br>55.1 | 4.53<br>4.7 | 7.05<br>7.1 | Mpt: 112—114°C |
| 22B | 2,4-dichloro-phenyl | 3-pyridyl | H | —COOEt | $-OC(=O)NHCH_3$ | Calc.<br>Found. | 54.41<br>54.4 | 4.53<br>5.3 | 7.05<br>6.7 | Glass |
| 23A | 3-pyridyl | 2,4-di-chlorophenyl | H | $COOCH_3$ | $CH_3C(=O)OCH_2CH_2S-$ | Calc.<br>Found. | 53.27<br>53.1 | 4.44<br>4.6 | 3.27<br>3.6 | Mpt: 83—85°C |
| 23B | 3-pyridyl | 2,4-di-chlorophenyl | H | $COOCH_3$ | $CH_3C(=O)OCH_2CH_2S-$ | Calc.<br>Found. | 53.27<br>53.1 | 4.44<br>4.7 | 3.27<br>3.2 | oil |
| 24A | 3-pyridyl | 2,4-di-chlorophenyl | H | $COOCH_3$ | $CH_3NHC(=O)OCH_2CH_2S-$ | Calc.<br>Found. | 51.47<br>51.3 | 4.51<br>4.6 | 6.32<br>6.2 | Mpt: 107—109°C |
| 24B | 3-pyridyl | 2,4-di-chlorophenyl | H | $COOCH_3$ | $CH_3NHC(=O)OCH_2CH_2S-$ | Calc.<br>Found. | 51.47<br>51.5 | 4.51<br>4.6 | 6.32<br>6.1 | oil |
| 25 | 3-pyridyl | 2,4-di-chlorophenyl | H | $COOCH_3$ | $ClCH_2CH_2S-$ | Calc.<br>Found. | 50.43<br>50.3 | 3.96<br>4.0 | 3.4<br>3.4 | Mpt: 84—86°C |
| 26 | 3-pyridyl | 2,4-di-chlorophenyl | H | $COOCH_3$ | $HC(=O)OCH_2CH_2S-$ | Calc.<br>Found. | 52.17<br>52.2 | 4.11<br>4.2 | 3.38<br>3.3 | Mpt: 79—83 |

Example 15

(a) Activity against vine downy mildew (Plasmopara viticola; Pv.t)

The test is a translaminar protectant one using a foliar spray. The upper surfaces of leaves of whole vine plants are sprayed using a moving track sprayer. The track sprayer delivers 620 l/ha and the concentration of active material is calculated to give an application rate of 1 kg/ha. The lower surfaces of the leaves are then inoculated, up to 6 hours after treatment with the test compound, by spraying with an aqueous suspension containing $10^5$ zoosporangia/ml. The inoculated plants are kept for 24 hours in a high humidity compartment, 4 days at glasshouse ambient temperature and humidity and then returned for a further 24 hours to high humidity. Assessment is based on the percentage of leaf area covered by sporulation compared with that on control leaves.

(b) Activity against vine grey mould (Botrytis cinerea; B.c.).

The test is a direct eradicant one using a foliar spray. The under-surface of the detached vine leaves are inoculated by pipetting ten large drops of an aqueous suspension containing $5\times10^5$ conidia/ml on to them. The inoculated leaves kept uncovered overnight during which time the fungus has penetrated the leaf and a visible necrotic lesion may be apparent where the drop was made. The infected regions are sprayed directly with a dosage of 1 kg of active material per hectare using a track sprayer as described under (a). When the spray has dried the leaves are covered with a petri dish and the disease allowed to develop under these humid conditions. The extent of the necrotic lesion beyond the original drop together with the degree of sporulation is compared with that on control leaves.

(c) Activity against potato late blight (Phytophtora infestans; P.i.p.)

The test measures the direct protectant activity of compounds applied as a foliar spray. Tomato plants, cultivar Ailsa Craig, 1—15 cms high, in monopots are used. The whole plant is sprayed at a dosage of 1 kilogram of active material per hectare using a track sprayer. The plant is then inoculated up to 6 hours after treatment with the test compound, by spraying with an aqueous suspension containing $5\times10^3$ zoosporangia/ml. The inoculated plants are kept in high humidity for 3 days. Assessment is based on comparison between the levels of disease on the treated and control plants.

(d) Activity against barley powdery mildew (Erysiphe graminis; E.g.)

The test measures the direct antisporulant activity of compounds applied as a foliar spray. For each compound about 40 barley seedlings are grown to the one-leaf stage in a plastic pot of sterile potting compost. Inoculation is effected by dusting the leaves with conidia of Erysiphe graminis, spp. hordei. 24 hours after inoculation the seedlings are sprayed with a solution of the compound in a mixture of acetone (50%), surfactant (0.04%) and water using a track sprayer as described under (a). The rate of application is equivalent to 1 kg of active material per hectare. Assessment of disease is made 5 days after treatment, when the overall level of sporulation on the treated plants is compared with that on control plants.

(e) Activity against apple powdery mildew (Podsophaera leucotrica; P.l.)

The test is a direct anti-sporulant one using a foliar spray. The upper surfaces of leaves of whole apple seedlings are inoculated by spraying with an aqueous suspension containing $10^5$ conidia/ml 2 days prior to treatment with the test compound. The inoculated plants are immediately dried and kept at glasshouse ambient temperatures and humidity prior to treatment. The plants are sprayed at a dosage of 1 kilogram of active material per hectare using a track sprayer. After drying the plants are returned to a compartment at ambient temperatures and humidity for up to 9 days, followed by assessment. Assessment is based on the percentage of the leaf area covered by sporulation compared with that on leaves of control plants.

(f) Activity against peanut leaf spot (Cercospora arachidicola; Ca)

The test is a direct eradicant one using a folar spray. The upper surfaces of the leaves of peanut plants (12—20 cms high, monopots) are inoculated by spraying with an aqueous suspension containing $10^5$ conidia/ml 4 hours prior to treatment with the test compound. The inoculated plants are kept at high humidity and then allowed to dry during the interval between inoculation and treatment by spraying at a dosage of 1 kg of active material per hectare using a track sprayer. After spraying the plants are moved to a humid compartment at 25—28°C for a further period of up to 10 days. Assessment is based on a comparison between the levels of disease on the treated and control plants.

The extent of disease control achieved in these tests is expressed as a control rating in Table II below; greater than 80% disease control is given the rating 2 after the test; control of between 50 and 80% is given the rating 1 after the test.

TABLE II

| Compound of Example No. | Greater than 50% disease control achieved in the below indicated tests |
|---|---|
| 1A | Pip (1) Eg (2) Pl (2) |
| 1B | Bc (1) Eg (2) Pl (1) |
| 2 | Pip (1) Pl (2) Ca (1) |
| 3A | Pip (1) |
| 4A | Pip (1) |
| 5B | Bc (2) Eg (2) |
| 5C | Eg (2) |
| 6A | Bc (1) Pip (1) Eg (2) |
| 6b | Pip(1) Eg (2) |
| 7 | Pvt (1) Pip (1) Pl (2) |
| 8A | Eg (2) Pl (1) |
| 8B | Eg (2) |
| 9A | Pl (2) |
| 9B | Pl (2) |
| 10A | Eg (1) |
| 10B | Eg (1) |
| 11A | Eg (2) |
| 11B | Eg (1) |
| 12A | Eg (2) |
| 12B | Eg (2) Pl (1) |
| 13A | Eg (2) Pl (2) |
| 13B | Eg (2) |
| 13C | Eg (2) |
| 14 | Pvt (1) Pip (1) Eg (2) |
| 16A | Pvt (1) Bc (1) Eg (2) Pl (2) |
| 18 | Eg (2) Pl (2) |
| 19 | Eg (1) Pl (2) |
| 20A | Pl (1) |
| 20B | Pl (2) |
| 21A | Pl (2) |

TABLE II (contd.)

| Compound of Example No. | Greater than 50% disease control achieved in the below indicated tests |
|---|---|
| 21B | Pl (1) |
| 22A | Eg (1) |
| 22B | Pl (1) |
| 23A | Eg (1) Pl (2) |
| 23B | Pl (2) |
| 24A | Eg (1) Pl (2) |
| 24B | Eg (1) Pl (2) |
| 25 | Pvt (1) Bc (1) Eg (1) Pl (2) |
| 26 | Eg (1) Pl (2) |

**Claims**

1. A compound of the general formula (I)

$$Ar^1-\underset{R^3}{\overset{R^1}{C}}-\underset{H}{\overset{R^2}{C}}-Ar^2 \qquad (I)$$

or an N-oxide, salt or metal salt complex thereof, wherein one of $Ar^1$ and $Ar^2$ represents an unsubstituted or substituted 6 membered heteroaromatic ring containing 1 or 2 nitrogen atoms and the other of $Ar^1$ and $Ar^2$ represents an unsubstituted or substituted 5 or 6 membered heterocyclic ring having 1 or 2 heteroatoms selected from nitrogen, oxygen and sulphur atoms or an unsubstituted or substituted phenyl group; $R^1$ represents a hydrogen atom or an unsubstituted or substituted $C_{1-6}$ alkyl group; $R^2$ represents a cyano group, a —COOH group or a $C_{1-6}$ alkyl ester or a $C_{1-6}$ alkyl thioester thereof, or a group

$$-C(=O)-N(R^4)(R^5) \quad \text{or} \quad -C(=O)-Y$$

wherein each of $R^4$ and $R^5$ independently represents a hydrogen atom or an unsubstituted or substituted $C_{1-6}$ alkyl or phenyl group or $R^4$ and $R^5$ together with the interjacent nitrogen atom represent a 5 or 6 membered heterocyclic ring containing up to one heteroatom selected from oxygen and nitrogen atoms in addition to the interjacent nitrogen atom and Y represents a halogen atom; $R^3$ represents a cyano group, a COOH group or a $C_{1-6}$ alkyl ester or a $C_{1-6}$ alkyl thioester thereof, a hydroxy or thio group, or a group

$$-OR^6,\ -SR^6,\ -\overset{O}{\overset{\|}{S}}-R^6,\ -\underset{O}{\underset{\|}{\overset{O}{\overset{\|}{S}}}}-R^6,\ -OR^7,\ -SR^7,\ -OCONR^8R^9 \text{ or } -NR^8R^9$$

wherein $R^6$ represents an unsubstituted or substituted $C_{1-6}$ alkyl or phenyl group, $R^7$ represents a $C_{1-6}$ alkanoyl, benzoyl or toluyl group, and $R^8$ and $R^9$ are as defined above for $R^4$ and $R^5$, and wherein the substituents of substituted aliphatic groups are selected from halogen atoms, $C_{1-6}$ alkoxy, $C_{1-6}$ alkylthio, cyano, carboxy, $C_{1-6}$ alkoxycarbonyl formyloxy, methylcarbonyloxy methylaminocarbonyloxy, hydroxy, amino and $C_{3-6}$ cycloalkyl groups and unsubstituted or substituted phenyl or phenoxy groups; and the

substituents of substituted phenyl, phenoxy and heterocyclic groups are selected from halogen atoms, hydroxy, methylenedioxy, amino, cyano, $C_{1-6}$ alkylsulphonyl and nitro groups and unsubstituted or substituted $C_{1-6}$ alkyl, $C_{2-6}$ alkenyl, $C_{2-6}$ alkynyl, $C_{1-6}$ alkoxy, $C_{1-6}$ alkylthio, $C_{1-6}$ alkoxycarbonyl, phenyl and phenoxy groups; with the proviso that, when $Ar^2$ represents an unsubstituted phenyl group, $R^1$ represents a hydrogen atom, $R^2$ represents $—CONH_2$ and $R^3$ represents $—NH_2$, then $Ar^1$ does not represent an unsubstituted 2-, 3- or 4-pyridyl group.

2. A compound as claimed in Claim 1 wherein one of $Ar^1$ and $Ar^2$ represents an unsubstituted pyrazine group or an unsubstituted pyridyl group and the other of $Ar^1$ and $Ar^2$ represents an unsubstituted pyridyl group or an unsubstituted or substituted phenyl group.

3. A compound as claimed in Claim 2 wherein one of $Ar^1$ and $Ar^2$ represents an unsubstituted 3-pyridyl group and the other of $Ar^1$ and $Ar^2$ represents an unsubstituted 3-pyridyl group or a halo-substituted phenyl group.

4. A compound as claimed in Claim 3 wherein one of $Ar^1$ and $Ar^2$ represents an unsubstituted 3-pyridyl group and the other of $Ar^1$ and $Ar^2$ represents a dichloro-substituted phenyl group.

5. A compound as claimed in any one of the preceding claims wherein $R^1$ represents a hydrogen atom.

6. A compound as claimed in any one of the preceding claims, wherein $R^2$ represents a cyano group, a —COOH group or a $C_{1-6}$ alkyl ester or a $C_{1-6}$ alkyl thioester thereof, or a group

$$—C(=O)—N(R^4)(R^5) \quad \text{or} \quad —C(=O)—Cl$$

wherein each of $R^4$ and $R^5$ independently represents a hydrogen atom, an unsubstituted $C_{1-6}$ alkyl group or an unsubstituted or substituted phenyl group, or $R^4$ and $R^5$ together with the interjacent nitrogen atom represent a pyrrolidine or piperidine ring.

7. A compound as claimed in Claim 6 wherein $R^2$ represents a group —COOH or a $C_{1-6}$ alkyl ester or a $C_{1-6}$ alkyl thioester thereof.

8. A compound as claimed in any one of the preceding claims wherein $R^3$ represents a cyano group, a hydroxy group or a group

$$—OR^6,\ —SR^6,\ —S(=O)—R^6,\ —S(=O)_2—R^6,\ —OR^7,\ —SR^7 \text{ or } —NR^8R^9$$

wherein $R^6$ represents an unsubstituted or substituted $C_{1-6}$ alkyl group or an unsubstituted or substituted phenyl group; $R^7$ represents $C_{1-6}$ alkanoyl group benzoyl or toluyl group; and each of $R^8$ and $R^9$ is as defined in Claim 6 for $R^4$ and $R^5$.

9. A compound as claimed in Claim 8 wherein $R^3$ represents a hydroxy group or a group

$$—OR^6,\ —SR^6, \text{ or } —S(=O)_2—R^6$$

wherein $R^6$ represents a $C_{1-4}$ alkyl group or a phenyl group which is unsubstituted or substituted by one or more halogen atoms.

10. A process for the preparation of a compound as claimed in Claim 1, which comprises reacting a compound of the general formula

$$Ar^1R^1C{=}CR^{10}Ar^2 \qquad (II)$$

in which $Ar^1$, $R^1$ and $Ar^2$ have the meanings given in Claim 1, and $R^{10}$ represents a —COOH group or a $C_{1-6}$ alkyl ester or a $C_{1-6}$ alkyl thioester thereof, with a compound of the general formula $HR^{11}$, in which $R^{11}$ represents a cyano, hydroxy or thio group or a group of formula $—OR^6$, $—SR^6$ or $—NR^8R^9$ where $R^6$, $R^8$ and $R^9$ have the meanings given in Claim 1; and if desired, converting the resulting compound as claimed in Claim 1 into any other compound as claimed in Claim 1.

11. A fungicidal composition which comprises a compound as claimed in any one of Claims 1 to 9 in association with at least one carrier.

12. A composition as claimed in Claim 11 which comprises at least two carriers, at least one of which is a surface active agent.

13. A method of combating fungus at a locus which comprises applying to the locus a compound as claimed in any one of Claims 1 to 9 or a composition as claimed in Claim 11 or 12.

**Patentansprüche**

1. Verbindung der allgemeinen Formel

$$Ar^1-\underset{R^3}{\overset{R^1}{C}}-\underset{H}{\overset{R^2}{C}}-Ar^2 \qquad (I)$$

oder ein N-Oxid, Salz oder Metallsalz-Komplex davon, wobei einer der Reste $Ar^1$ und $Ar^2$ einen unsubstituieren oder substituierten 6-gliedrigen heteroaromatischen Rest mit 1 oder 2 Stickstoffatomen und der andere der Reste $Ar^1$ und $Ar^2$ einen unsubstituierten oder substituierten 5- oder 6-gliedrigen heterocyclischen Ring mit 1 oder 2 Heteroatomen, ausgewählt aus Stickstoff-, Sauerstoff- und Schwefelatomen, oder eine unsubstituierte oder substituierte Phenylgruppe bedeutet, $R^1$ ein Wasserstoffatom oder eine unsubstituierte oder substituierte $C_{1-6}$-Alkylgruppe bedeutet, $R^2$ eine Cyanogruppe, eine Gruppe —COOH oder einen $C_{1-6}$-Alkylester oder einen $C_{1-6}$-Alkylthioester davon bedeutet oder eine Gruppe

$$-C(=O)-N(R^4)(R^5) \quad \text{oder} \quad -C(=O)-Cl$$

wobei $R^4$ und $R^5$ jeweils unabhängig ein Wassestoffatom oder eine unsubstituierte oder substituierte $C_{1-6}$-Alkyl- oder Phenylgruppe oder $R^4$ und $R^5$ zusammen mit den dazwischenliegenden Stickstoffatom einen 5- oder 6-gliedrigen heterocyclischen Ring, enthaltend bis zu 1 Heteroatom, ausgewählt aus Sauerstoff- und Stickstoffatomen, zusätzlich zu dem dazwischenliegenden Stickstoffatom bedeuten, und Y ein Halogenatom ist, $R^3$ eine Cyanogruppe, eine Gruppe —COOH oder einen $C_{1-6}$-Alkylester oder einen $C_{1-6}$-Alkylthioester davon, eine Hydroxy- oder Thiogruppe oder eine Gruppe

$$-OR^6,\ -SR^6,\ -S(=O)-R^6,\ -S(=O)_2-R^6,\ -OR^7,\ -SR^7,\ -COONR^8R^9 \text{ oder } NR^8R^9$$

bedeutet, wobei $R^6$ eine unsubstituierte oder substituierte $C_{1-6}$-Alkyl- oder eine Phenylgruppe, $R^7$ eine $C_{1-6}$-Alkanoyl- oder Benzoyl- oder Toluylgruppe bedeutet und $R^8$ und $R^9$ die obeh für $R^4$ und $R^5$ angegebene Bedeutung haben und wobei die Substituenten von substituierten aliphatischen Gruppen ausgewählt sind aus Halogenatomen, $C_{1-6}$-Alkoxy-, $C_{1-6}$-Alkylthio-, Cyano-, Carboxy-$C_{1-6}$-Alkoxycarbonyl, Formyloxy-, Methylcarbonyloxy-, Methylamino-, carbonyloxy-, Hydroxy-, Amino- und $C_{3-6}$-Cycloalkylgruppen und unsubstituierten oder substituierten Phenyl- oder Phenoxygruppen und die Substituenten von substituierten Phenyl-, Phenoxy- und hetercyclischen Gruppen ausgewählt sind aus Halogenatomen, Hydroxy-, Methylendioxy-, Amino-, Cyano-, $C_{1-6}$-Alkylsulfonyl- und Nitrogruppen und unsubstituierten oder substituierten $C_{1-6}$-Alkyl-, $C_{2-6}$-Alkenyl-, $C_{2-6}$-Alkinyl-, $C_{1-6}$-Alkoxy-, $C_{1-6}$-Alkylthio-, $C_{1-6}$-Alkoxycarbonyl-, Phenyl- und Phenoxygruppen, mit der Maßgabe, daß wenn $Ar^2$ eine unsubstituierte Phenylgruppe ist, $R^1$ ein Wasserstoffatom bedeutet, $R^2$ die Gruppe —$CONH_2$ bedeutet und $R^3$ —$NH_2$ bedeutet, $Ar^1$ keine unsubstituierte 2-, 3- oder 4-Pyridylgruppe ist.

2. Verbindung nach Anspruch 1, wobei einer der Reste $Ar^1$ und $Ar^2$ eine unsubstituierte Pyrazingruppe oder eine unsubstituierte Pyridylgruppe und der andere der Reste $Ar^1$ und $Ar^2$ eine unsubstituierte Pyridylgruppe oder eine unsubstituierte oder substituierte Phenylgruppe bedeutet.

3. Verbindung nach Anspruch 2, wobei einer der Reste $Ar^1$ und $Ar^2$ eine unsubstituierte 3-Pyridylgruppe und der andere der Reste $Ar^1$ und $Ar^2$ eine unsubstituierte 3-Pyridylgruppe oder eine Halogen-substituierte Phenylgruppe bedeutet.

4. Verbindung nach Anspruch 3, wobei einer der Reste $Ar^1$ und $Ar^2$ eine unsubstituierte 3-Pyridylgruppe und der andere der Reste, $Ar^1$ und $Ar^2$ eine Dichlor-substituierte Phenylgruppe bedeutet.

5. Verbindung nach einem der vorangehenden Ansprüche, wobei $R^1$ ein Wasserstoffatom ist.

6. Verbindung nach einem der vorangehenden Ansprüche, wobei $R^2$ eine Cyanogruppe, eine —COOH-Gruppe oder einen $C_{1-6}$-Alkylester oder einen $C_{1-6}$-Alkylthioester davon oder eine Gruppe

O
—C
R4
N
R5
oder
O
—C
Cl

bedeutet, wobei $R^4$ und $R^5$ jeweils unabhängig ein Wasserstoffatom, eine unsubstituierte $C_{1-6}$-Alkylgruppe oder eine unsubstituierte oder substituierte Phenylgruppe bedeuten oder $R^4$ und $R^5$ zusammen mit dem dazwischenliegenden Stickstoffatom einen Pyrrolidin- oder Piperidinring darstellen.

7. Verbindung nach Anspruch 6, wobei $R^2$ eine Gruppe —COOH oder einen $C_{1-6}$-Alkylester oder einen $C_{1-6}$-Alkylthioester davon bedeutet.

8. Verbindung nach einem der vorangehenden Ansprüche, wobei $R^3$ eine Cyanogruppe, eine Hydroxygruppe oder eine Gruppe

$$-OR^6,\ -SR^6,\ -\overset{O}{\overset{\|}{S}}-R^6,\ -\underset{\underset{O}{\|}}{\overset{\overset{O}{\|}}{S}}-R^6,\ -OR^7,\ -SR^7 \text{ oder } -NR^8R^9 \text{ ist,}$$

wobei $R^6$ eine unsubstituierte oder substituierte $C_{1-6}$-Alkylgruppe oder eine unsubstituierte oder substituierte Phenylgruppe ist, $R^7$ eine $C_{1-6}$-Alkanoylgruppe, eine Benzoyl- oder Toluylgruppe ist und $R^8$ und $R^9$ jeweils die in Anspruch 6 für $R^4$ und $R^5$ angegebene Bedeutung haben.

9. Verbindung nach Anspruch 8, wobei $R^3$ eine Hydroxygruppe oder eine Gruppe

$$-OR^6,\ -SR^6 \text{ oder } -\underset{\underset{O}{\|}}{\overset{\overset{O}{\|}}{S}}-R^6 \quad \text{bedeutet,}$$

wobei $R^6$ eine $C_{1-4}$-Alkylgruppe oder eine Phenylgruppe ist, die unsubstituiert oder durch ein oder mehrere Halogenatome substituiert ist.

10. Verfahren zur Herstellung einer Verbindung nach Anspruch 1, umfassend die Umsetzung einer Verbindung der allgemeinen Formel

$$Ar^1R^1C{=}CR^{10}Ar^2 \qquad \text{(II)}$$

in der $Ar^1$, $R^1$ und $Ar^2$ die in Anspruch 1 angegebene Bedeutung haben und $R^{10}$ eine Gruppe —COOH oder einen $C_{1-6}$-Alkylester oder einen $C_{1-6}$-Alkylthioester davon bedeutet, mit einer Verbindung der allgemeinen Formel $HR^{11}$, in der $R^{11}$ eine Cyano-, Hydroxy-, oder Thiogruppe oder eine Gruppe der Formel $-OR^6$, $-SR^6$ oder $-NR^8R^9$ ist, in der $R^6$, $R^8$ und $R^9$ die in Anspruch 1 angegebene Bedeutung haben, und gegebenenfalls Umwandlung der erhaltenen Verbindung nach Anspruch 1 in eine beliebige andere Verbindung nach Anspruch 1.

11. Fungizides Mittel, umfassend eine Verbindung nach einem der Ansprüche 1 bis 9, zusammen mit mindestens einem Träger.

12. Mittel nach Anspruch 11, umfassend mindestens zwei Träger von denen mindestens einen ein oberflächenaktives Mittel ist.

13. Verfahren zur Bekämpfung von Pilzen einer Stelle, umfassend das Aufbringen einer Verbindung nach einem der Ansprüche 1 bis 9 oder eines Mittels nach Anspruch 11 und 12 auf diese Stelle.

**Revendications**

1. Un composé de la formule générale (I)

$$Ar^1-\underset{\underset{R^3}{|}}{\overset{\overset{R^1}{|}}{C}}-\underset{\underset{H}{|}}{\overset{\overset{R^2}{|}}{C}}-Ar^2$$

ou un N-oxyde, un sel ou un complexe de sel de métal d'un tel composé, où un de $Ar^1$ et $Ar^2$ représente un noyau hétéro-aromatique hexagonal substitué ou non contenant 1 ou 2 atomes d'azote et l'autre de $Ar^1$ et $Ar^2$ représente un noyau hétérocyclique pentagonal ou hexagonal substitué ou non ayant 1 ou 2 hétéro-atomes choisis parmi des atomes d'azote, d'oxygène et de soufre ou un groupe phényle substitué ou non; $R^1$ représente un atome d'hydrogène ou un groupe alcoyle en $C_{1-6}$ substitué ou non; $R^2$ représente un groupe cyano, un groupe —COOH ou un ester de $C_{1-6}$ alcoyle ou un thioester de $C_{1-6}$ alcoyle correspondant, ou un groupe

$$-C(=O)-N(R^4)(R^5) \quad \text{ou} \quad -C(=O)-Cl$$

où chacun de $R^4$ et $R^5$ indépendamment représente un atome d'hydrogène ou un groupe alcoyle en $C_{1-6}$ ou phényle substitué ou non ou $R^4$ et $R^5$ en même temps que l'atome d'azote situé entre eux représentent un noyau hétérocyclique pentagonal ou hexagonal contenant au maximum un hétéro-atome choisi parmi des atomes d'oxygène et d'azote en plus de l'atome d'azote intermédiaire et Y représente un atome d'halogène; $R^3$ représente un groupe cyano, un groupe COOH ou un ester d'alcoyle en $C_{1-6}$ ou un thioester d'alcoyle en $C_{1-6}$ correspondant, un groupe hydroxy ou thio ou un groupe

$$-OR^6,\ -SR^6,\ -S(=O)-R^6,\ -S(=O)_2-R^6,\ -OR^7,\ -SR^7,\ -OCONR^8R^9 \ \text{ou}\ -NR^8R^9$$

où $R^6$ représente un groupe alcoyle en $C_{1-6}$ ou phényle substitué ou non, $R^7$ représente un groupe alcoyle en $C_{1-6}$ ou benzoyle ou toluyle et $R^8$ et $R^9$ sont tels que définis ci-dessus pour $R^4$ et $R^5$, et les substituants de groupes aliphatiques substitués sont choisi parmi des atomes d'halogènes, des groupes $C_{1-6}$ alcoxy, $C_{1-6}$-alcoylthio, cyano, carboxy, $C_{1-6}$ alcoxycarbonyle, formyloxy, méthylcarbonyloxy, méthylaminocarbonyloxy, hydroxy, amino et $C_{3-6}$ cycloalcoyle et des groupes phényle ou phénoxy substitué ou non; et les substituants des groupes phényle, phénoxy et hétérocycliques substitués sont choisis parmi des atomes d'halogènes, des groupes hydroxy, méthylènedioxy, amino, cyano, $C_{1-6}$ alcoylsulfonyle et nitro et des groupes $C_{1-6}$ alcoyle, $C_{2-6}$ alcényle, $C_{2-6}$ alcynyle, $C_{1-6}$ alcoxy, $C_{1-6}$ alcoylthio, $C_{1-6}$ alcoxycarbonyle, phényle et phénoxy substitués ou non; avec la condition que, lorsque $Ar^2$ représente un groupe phényle non substitué, $R^1$ représente un atome d'hydrogène, $R^2$ représente —$CONH_2$ et $R^3$ représente —$NH_2$, alors $Ar^1$ ne représente pas un groupe 2-, 3- ou 4-pyridyle non substitué.

2. Un composé selon la revendication 1, dans lequel un de $Ar^1$ et $Ar^2$ représente un groupe pyrazine non substitué ou un groupe pyridyle non substitué et l'autre de $Ar^1$ et $Ar^2$ représente un groupe pyridyle non substitué ou un groupe phényle non substitué.

3. Un composé selon la revendication 2, dans lequel un de $Ar^1$ et $Ar^2$ représente un groupe 3-pyridyle non substitué et l'autre de $Ar^1$ et $Ar^2$ représente un groupe 3-pyridyle non substitué ou un groupe phényle halogéné.

4. Un composé selon la revendication 3, dans lequel un de $Ar^1$ et $Ar^2$ représente un groupe 3-pyridyle non substitué et l'autre de $Ar^1$ et $Ar^2$ représente un groupe phényle dichloré.

5. Un composé selon l'une quelconque des revendications précédentes, dans lequel $R^1$ représente un atome d'hydrogène.

6. Un composé selon l'une quelconque des revendications précédentes, dans lequel $R^2$ représente un groupe cyano, un groupe —COOH ou un ester de $C_{1-6}$ alcoyle ou un thioester de $C_{1-6}$ alcoyle correspondant, ou un groupe

$$-C(=O)-N(R^4)(R^5) \quad \text{ou} \quad -C(=O)-Cl$$

où chacun de $R^4$ et $R^5$ indépendamment représente un atome d'hydrogène, un groupe alcoyle en $C_{1-6}$ non substitué ou un groupe phényle substitué ou non, ou $R^4$ et $R^5$ en même temps que l'atome d'azote situé entre eux représentent un noyau de pyrrolidine ou de pipéridine.

7. Un composé selon la revendication 6, dans lequel $R^2$ représente un groupe —COOH ou un ester d'alcoyle en $C_{1-6}$ ou un thioester d'alcoyle en $C_{1-6}$ correspondant.

8. Un composé selon l'une quelconque des revendications précédentes, dans lequel $R^3$ représente un groupe cyano, un groupe hydroxy ou un groupe

$$-OR^6,\ -SR^6,\ -\overset{\displaystyle O}{\overset{\|}{S}}-R^6,\ -\underset{\underset{\displaystyle O}{\|}}{\overset{\overset{\displaystyle O}{\|}}{S}}-R^6,\ -OR^7,\ -SR^7 \text{ ou } -NR^8R^9$$

où $R^6$ représente un groupe alcoyle en $C_{1-6}$ substitué ou non ou un groupe phényle substitué ou non; $R^7$ représente un groupe alcanoyle en $C_{1-6}$, benzoyle ou toluyle; et chacun de $R^8$ et $R^9$ est tel que défini dans la revendication 6 pour $R^4$ et $R^5$.

9. Un composé selon la revendication 8, dans lequel $R^3$ représente kmugroupe hydroxy ou un groupe

$$-OR^6,\ -SR^6 \text{ ou } -\underset{\underset{\displaystyle O}{\|}}{\overset{\overset{\displaystyle O}{\|}}{S}}-R^6$$

où $R^6$ représente un groupe alcoyle en $C_{1-6}$ ou un groupe phényle qui n'est pas substitué ou est substitué par un ou plusieurs atomes d'halogènes.

10. Un procédé de préparation d'un composé tel que revendiqué dans la revendication 1, qui comprend la réaction d'un composé de la formule générale

$$Ar^1R^1C{=}CR^{10}Ar^2 \qquad (II)$$

où $Ar^1$, $R^1$ et $Ar^2$ ont les significations indiquées dans la revendication 1 et $R^{10}$ représente un groupe —COOH où un ester d'alcoyle en $C_{1-6}$ ou un thioester d'alcoyle en $C_{1-6}$ correspondant, avec un composé de la formule générale $HR^{11}$, où $R^{11}$ représente un groupe cyano, hydroxy ou thio ou un groupe de formule $-OR^6$, $-SR^6$ ou $-NR^8R^9$ où $R^6$, $R^8$ et $R^9$ ont les significations indiquées dans la revendication 1 et, si on le désire, la conversion du composé selon la revendication 1 résultant et un autre composé quelconque selon la revendication 1.

11. Une composition fongicide qui comprend un composé selon l'une quelconque des revendications 1 à 4, en association avec au moins un véhicule.

12. Une composition selon la revendication 11 qui comprend au moins deux véhicules, dont au moins un est un agent tensio-actif.

13. Un procédé de lutte contre les champignons en un lieu, qui comprend l'application à ce lieu d'un composé selon l'une quelconque des revendications 1 à 9, ou d'une composition selon la revendication 11 ou 12.